(19) **Europäisches Patentamt / European Patent Office / Office européen des brevets**

(11) **EP 4 640 184 A1**

(12) **EUROPEAN PATENT APPLICATION**
published in accordance with Art. 153(4) EPC

(43) Date of publication:
**29.10.2025 Bulletin 2025/44**

(21) Application number: **23907037.8**

(22) Date of filing: **19.12.2023**

(51) International Patent Classification (IPC):
***A61F 2/16*** *(2006.01)*

(52) Cooperative Patent Classification (CPC):
**A61F 2/16**

(86) International application number:
**PCT/JP2023/045486**

(87) International publication number:
**WO 2024/135677 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.12.2022 JP 2022207693**
**31.03.2023 JP 2023058516**

(71) Applicant: **Nidek Co., Ltd.**
**Gamagori-shi, Aichi 443-0038 (JP)**

(72) Inventors:
- **MURASE Kyoko**
  **Gamagori-shi Aichi 443-0038 (JP)**
- **NAKAHATA Yoshihiro**
  **Gamagori-shi Aichi 443-0038 (JP)**
- **NAGASAKA Shinji**
  **Gamagori-shi Aichi 443-0038 (JP)**

(74) Representative: **Viering, Jentschura & Partner mbB**
**Patent- und Rechtsanwälte**
**Am Brauhaus 8**
**01099 Dresden (DE)**

(54) **INTRAOCULAR LENS**

(57) The intraocular lens (1) comprises a disc-shaped lens section (2). At least one of the front and rear surfaces of the lens section (2) is formed with three or more segmental regions (20) having mutually different refractive powers. The segmental regions (20) include a distance region (20A) with the smallest refractive power, a near region (20B) with the largest refractive power, and an intermediate region (20C) with a refractive power between that of the distance region (20A) and the near region (20B). The multiple segmental regions (20) radiate outwardly from the center of the lens section (2).

**FIG. 5**

**EP 4 640 184 A1**

**Description**

[Technical Field]

The present disclosure relates to an intraocular lens that is inserted into an eye.

[Background Art]

As intraocular lenses inserted into the eye, multifocal intraocular lenses have been known, which distribute incident light to the lens section into multiple focal points for focusing. Multifocal intraocular lenses can provide the wearer with a pseudo-accommodative power. Multifocal intraocular lenses include refractive-type lenses, in which multiple regions with different refractive powers are formed in the optical part, and diffractive-type lenses that utilize the phenomenon of light diffraction. Refractive-type multifocal intraocular lenses have the advantage of less light loss reaching the retina compared to diffractive-type multifocal intraocular lenses.

As described in Patent Document 1, in conventional refractive-type multifocal intraocular lenses, it is common for the lens section to have multiple regions with different refractive powers arranged concentrically. When multiple regions in the lens section are arranged concentrically, if the wearer's pupil becomes small, only the regions near the center arranged on the concentric circles allow light to pass through, and light does not pass through the peripheral regions, making it difficult to achieve the multifocal effect.

In the multifocal intraocular lens described in Patent Document 2, the disc-shaped optical part is divided into multiple regions by boundary lines extending from the periphery toward the geometric center, and in each divided region, a microprism group is formed in a different pattern, thereby imparting different refractive powers to each region.

[Prior Art Literature]

[Patent Documents]

Patent Document 1: JP 2010-082290 A
Patent Document 2: JP 2010-125292 A

[Summary of Invention]

A first aspect of the present invention will be described. The multifocal intraocular lens described in Patent Document 2 faces difficulties in processing microprism groups with high precision, and there is also the problem that the microprism groups are easily damaged during insertion into the eye. Furthermore, part of the light passing through the microprism groups may scatter in unintended directions, reducing the amount of light focused on the retina, making it difficult to obtain a good visual field.

A second aspect of the present invention will be described. In intraocular lenses where multiple regions in the lens section are arranged concentrically, as described in Patent Document 1, if the wearer's pupil becomes small, only the regions near the center arranged concentrically allow light to pass through. As a result, light does not pass through the peripheral regions, making it difficult to achieve the multifocal effect. Therefore, in the intraocular lens described in Patent Document 1, the wearer may not obtain a good visual field through multifocality. Also, even in Patent Document 2, it cannot be said that sufficient consideration has been given to providing the wearer with a good visual field through multifocality. In other words, with conventional intraocular lenses, it has been difficult to appropriately provide the wearer with a good visual field through multifocality regardless of pupil size.

A typical object of the present disclosure is to provide an intraocular lens that allows for a better visual field.

A typical embodiment of the intraocular lens provided by the present disclosure is an intraocular lens including: a disc-shaped lens section having a front surface and a rear surface. At least one of the front and rear surfaces of the lens section is formed with three or more segmental regions having mutually different refractive powers. The three or more segmental regions include: a distance region with a smallest refractive power; a near region with a largest refractive power; and an intermediate region with a refractive power between the smallest refractive power and the largest refractive power. The three or more segmental regions radiate outwardly from a center of the lens section.

According to the intraocular lens of the present disclosure, it becomes easier to obtain a better visual field.

A first aspect of the intraocular lens of the present disclosure will be described. The intraocular lens exemplified in the present disclosure comprises a disc-shaped lens section. At least one of the front and rear surfaces of the lens section is formed with three or more segmental regions having mutually different refractive powers. The multiple segmental regions include a distance region with the smallest refractive power, a near region with the largest refractive power, and an intermediate region with a refractive power between that of the distance and near regions. The multiple segmental regions radiate outwardly from the center of the lens section.

In the intraocular lens exemplified in the present disclosure, the multiple segmental regions with mutually different refractive powers radiate outwardly from the center of the lens section. That is, the multiple segmental regions are arranged in a circumferential direction around the center of the lens section. Therefore, unlike intraocular lenses with multiple regions arranged concentrically, even if the wearer's pupil becomes small, light can easily pass through all of the multiple segmental regions.

The multiple segmental regions may have different refractive powers due to mutually different radii of curvature. In this case, compared to forming microprism groups or the like in the segmental regions, the amount of light scattered in unintended directions is reduced. As a result, loss of light reaching the retina is less likely to occur, making it easier to obtain a better visual field. Furthermore, the lens is easier to process with high precision, and the risk of damage during insertion into the eye is also low.

The multiple segmental regions may radiate outwardly from a single reference point at the center of the lens section. In other words, the straight ends of each segmental region extending from the center of the lens section outward may all pass through the same reference point. In this case, even if the wearer's pupil becomes small, the amount of light passing through each segmental region is more easily ensured compared to the case where a certain region (e.g., a circular region) is separately formed at the center of the lens section. As a result, regardless of pupil size, the multifocal effect is more easily achieved, making it easier for the wearer to have a good visual field.

The lens surfaces of each segmental region may be spherical or aspherical. Within each segmental region, the refractive power is substantially constant. That is, if a transition region is provided on the lens surface, the refractive power changes within the transition region, whereas in the segmental region, the refractive power is substantially constant.

A transition region may be formed between adjacent segmental regions of the multiple segmental regions in the lens section. The transition region may smoothly connect the ends of the adjacent segmental regions by continuously changing the radius of curvature of the transition region from the end of one segmental region to the end of the other. If no transition region is formed, steps or the like may occur at the boundaries of adjacent segmental regions, which can cause light to be diffusely reflected and degrade the visual field (e.g., halo or glare). In contrast, forming a transition region between adjacent segmental regions reduces the influence of such diffuse reflection. Furthermore, when a transition region is formed, the refractive power in the transition region smoothly transitions from the refractive power of one adjacent segmental region to that of the other. As a result, in addition to the refractive powers of each segmental region, the lens section is also provided with refractive powers between those of the segmental regions. Thus, forming a transition region in the lens section appropriately provides the effect of extended depth of focus (EDOF) in multifocal intraocular lenses.

Both the segmental regions and the transition regions may refract light entering the lens section in a direction parallel to the optical axis toward the optical axis. In this case, the amount of light passing through the lens section but not reaching the retina is reduced, making it easier to obtain a better visual field. It is also possible to manufacture the intraocular lens without forming a transition region in the lens section. Even in this case, a better visual field is more easily obtained compared to forming microprism groups or the like in the segmental regions.

The central angle of the transition region radiating outward from the center of the lens section may be 5 degrees or more and 30 degrees or less. In this case, both the multifocal effect and the extended depth of focus effect from the multiple segmental regions are more easily achieved.

The central angle of the transition region may be 15 degrees or more and 20 degrees or less. In this case, both the multifocal effect and the extended depth of focus effect from the multiple segmental regions are even more easily achieved.

The central angles of the distance region, near region, and intermediate region radiating outward from the center of the lens section may be such that the distance region has the largest central angle and the intermediate region has the smallest angle. In this case, both distance vision by the distance region and near vision by the near region are more easily obtained with extended depth of focus.

The central angle of the distance region may be 140 degrees or more, the near region 90 degrees or more, and the intermediate region 30 degrees or more. In this case, both distance vision by the distance region and near vision by the near region are more easily obtained with extended depth of focus.

The refractive power of the near region may be +2.5 D or more and +4.0 D or less, and the refractive power of the intermediate region may be +1.0 D or more and +2.5 D or less. In this case, both the multifocal effect and the extended depth of focus effect from the multiple segmental regions are more easily achieved.

The refractive power of the near region may be +3.0 D or more and +3.5 D or less, and the refractive power of the intermediate region may be +1.5 D or more and +2.0 D or less. In this case, both the multifocal effect and the extended depth of focus effect are even more easily achieved.

At least one of the front and rear surfaces of the lens section may be formed with a toric surface to correct the astigmatism of the user (the wearer). In this case, an intraocular lens is provided that not only enables both distance and near vision but also corrects the wearer's astigmatism.

The multiple segmental regions may be formed on the front surface of the lens (i.e., the surface facing the front of the eye (corneal side) when implanted). In this case, the rear surface of the lens is more likely to be smooth, suppressing the entry of cells between the rear surface and the posterior capsule of the eye, thereby reducing the occurrence of posterior capsule opacification. It is also possible for both the multiple segmental regions and the toric surface to be formed on the front surface. In this case, even if a toric surface is formed, posterior capsule opacification is more easily suppressed. However, it is also possible for at least one of the segmental regions and the toric surface to be formed on the rear surface. The surfaces on which the segmental regions and the toric surface are formed may be the same or different.

The MTF curve at a spatial frequency of 50 lp/mm may have a distance maximum, a near maximum, and an intermediate maximum. The distance maximum is the maximum value in the range providing distance vision, the near maximum is the maximum value in the range providing near vision, and the intermediate maximum is the maximum value in the range between the distance and near maxima. Each of the distance, near, and intermediate maxima may be 0.10 or more.

In the intraocular lens exemplified in the present disclosure, the multiple segmental regions with mutually different refractive powers radiate outwardly from the center of the lens section. Therefore, unlike intraocular lenses with multiple regions arranged concentrically, even if the wearer's pupil becomes small, light can easily pass through all of the multiple segmental regions. Furthermore, if the MTF values at the distance, near, and intermediate maxima at a spatial frequency of 50 lp/mm are each 0.10 or more, good vision at distance, near, and intermediate ranges is more easily obtained regardless of pupil size.

In the MTF curve at a spatial frequency of 50 lp/mm, the difference between the smaller of the near and intermediate maxima and the minimum value between the near and intermediate maxima may be 0.10 or less. In this case, it is less likely for there to be a range of reduced vision between near and intermediate distances. In other words, good vision is more easily obtained overall from near to intermediate distances. Therefore, good vision at distance, near, and intermediate ranges is even more easily obtained regardless of pupil size.

The difference between the smaller of the near and intermediate maxima and the minimum value between the near and intermediate maxima in the MTF curve at a spatial frequency of 50 lp/mm may be 0.08 or less. More preferably, this difference may be 0.05 or less. In this case, vision from near to intermediate distances is even more easily obtained.

In the MTF curve, the near maximum may be greater than the intermediate maximum. In this case, contrast at the frequently viewed near distance is higher, making it easier to obtain a better visual field.

However, it is also possible for the near maximum to be less than or equal to the intermediate maximum. Even in this case, good vision at distance, near, and intermediate ranges is obtained regardless of pupil size.

In the MTF curve at a spatial frequency of 50 lp/mm, the difference between the near and intermediate maxima may be 0.05 or less. In this case, good vision is more easily obtained at both near and intermediate distances. Therefore, good vision at distance, near, and intermediate ranges is even more easily obtained regardless of pupil size.

If the refractive power of the near region is S and that of the intermediate region is M, the conditions $S \geq +2.75\,D$ and $M < S \leq (M + 1.4\,D)$ may both be satisfied. That is, the difference between the refractive powers of the near and intermediate regions may be 1.4 D or less. Through repeated trial and error and simulation, the inventors have newly found that bringing the difference between S and M to 1.4 D or less, compared to making it greater than 1.4 D, results in a synergistic effect of increasing the intermediate maximum in the MTF curve as well as raising the overall MTF values from intermediate to near distances. Therefore, by designing the intraocular lens to satisfy $S \geq +2.75\,D$ and $M < S \leq (M + 1.4\,D)$, good vision at distance, near, and intermediate ranges is even more easily obtained regardless of pupil size.

The intraocular lens may satisfy $S \geq +2.75\,D$ and $M < S \leq (M + 1.35\,D)$. That is, the difference between the refractive powers of the near and intermediate regions may be 1.35 D or less. In this case, the effect of increasing the intermediate maximum in the MTF curve and the effect of raising the overall MTF values from intermediate to near distances are even more easily obtained.

If the refractive power of the near region is S and that of the intermediate region is M, the conditions $S \geq +2.75\,D$ and $(M + 1.1\,D) \leq S \leq (M + 1.4\,D)$ may both be satisfied. As described above, bringing the difference between S and M to 1.4 D or less yields a useful synergistic effect. On the other hand, if the difference is made too small, the multifocal effect for obtaining good vision at distance, near, and intermediate ranges is reduced. Therefore, by designing the intraocular lens to satisfy $S \geq +2.75\,D$ and $(M + 1.1\,D) \leq S \leq (M + 1.4\,D)$, good vision at distance, near, and intermediate ranges is even more easily obtained through the multifocal effect regardless of pupil size.

The difference between the refractive powers of the near and intermediate regions may be 1.15 D or more. In this case, good vision at distance, near, and intermediate ranges is even more easily obtained through the multifocal effect.

Regardless of whether the above MTF value conditions are met, it is also possible to design the intraocular lens to satisfy $S \geq +2.75\,D$ and $M < S \leq (M + 1.4\,D)$. Even in this case, good vision at distance, near, and intermediate ranges is even more easily obtained regardless of pupil size by designing the intraocular lens to satisfy $S \geq +2.75\,D$ and $M < S \leq (M + 1.4\,D)$.

A second aspect of the intraocular lens of the present disclosure will be described. The intraocular lens exemplified in the present disclosure is deformable and is inserted into the patient's eye through the passage inside the intraocular lens insertion instrument from the insertion opening at the front end. The intraocular lens insertion instrument comprises an extrusion member and a nozzle. The extrusion member moves forward along the extrusion axis, which is the axis of the passage, to push the intraocular lens forward. The passage area of the nozzle is formed to decrease toward the front side. The nozzle has an insertion opening at its front end, and as the intraocular lens is pushed forward through the passage by the extrusion member, the intraocular lens is folded and then discharged from the insertion opening. The intraocular lens comprises a substantially disc-shaped lens section. A virtual cross-section in any direction that passes through the geometric center of the lens section and is perpendicular to the lens surface is assumed. In the intraocular lens of the present disclosure, the cross-sectional area of the lens section in the virtual cross-section varies depending on the direction. When the intraocular lens is folded by the nozzle, the cross-sectional area of the lens section in the first cross-section (perpendicular to the extrusion axis) is equal to or less than the cross-sectional area in other directions.

When the intraocular lens of the present disclosure is pushed out through the passage of the nozzle at an appropriate angle to the extrusion axis, the maximum value of the cross-sectional area in the direction perpendicular to the extrusion axis (i.e., the maximum cross-sectional area in the folded state in the first cross-section) is reduced. Therefore, the intraocular lens of the present disclosure can be folded small inside the passage of the nozzle.

The sum of the refractive powers along a pair of lines extending from the geometric center along the virtual cross-section to the periphery in both directions is calculated for each direction. The intraocular lens may be designed so that the sum of the refractive powers along the pair of lines in the first cross-section is equal to or greater than the sum in other directions.

In the lens section, the radius of curvature at a part with a large refractive power is smaller than that at a part with a small refractive power. In other words, the curvature of the lens surface at a part with a large refractive power is steeper than that at a part with a small refractive power. Since it is optically undesirable to provide a step at the geometric center, the thickness at the geometric center in any virtual cross-section is the same. As a result, the thickness at the lens edge at a part with a large refractive power is smaller than that at a part with a small refractive power. Therefore, the greater the sum of the refractive powers along the pair of lines, the smaller the cross-sectional area in the virtual cross-section. Thus, by designing the intraocular lens so that the sum in the first cross-section is equal to or greater than that in other directions, the cross-sectional area in the first cross-section is appropriately reduced. As a result, the intraocular lens can be more easily folded small inside the passage of the nozzle.

The method for defining the structure of the lens section 2 in the first cross-section may also be changed. For example, the sum of the radius of curvature along a line extending from the geometric center to one outer circumference along a virtual cross-section and the radius of curvature along a line extending from the geometric center to the other outer circumference along the virtual cross-section is calculated for each of the virtual cross-sections defined by any direction. Then, the intraocular lens may be designed so that the sum of the radii of curvature along the lines along the first cross-section is equal to or less than the sum of the radii of curvature along any other cross-section. Even in this case, the cross-sectional area in the first cross-section is appropriately reduced.

If the intraocular lens is a toric intraocular lens for correcting astigmatism, the lens section has a strong principal meridian and a weak principal meridian. The strong principal meridian is the meridian with the smallest radius of curvature and the maximum refractive power, and the weak principal meridian is the meridian with the largest radius of curvature and the minimum refractive power. In the toric intraocular lens, the position of the first cross-section may be made to coincide with the strong principal meridian. Even in this case, the cross-sectional area in the first cross-section is appropriately reduced.

The intraocular lens may further comprise a pair of support sections. The pair of support sections extend outward from different parts of the lens edge and support the lens section inside the eye. The intraocular lens may be designed so that the cross-sectional area in the second cross-section (passing through the bases of the support sections) is equal to or greater than that in other directions.

The bases of the support sections are preferably connected to thicker parts of the lens edge for stable support. If connected to thinner parts, unintended deformation or damage may occur when the support section is bent during insertion. By designing the intraocular lens so that the cross-sectional area in the second cross-section is equal to or greater than that in other directions, the thickness at the connection points is increased, ensuring rigidity and reducing the risk of unintended deformation or damage, thereby facilitating proper placement in the eye. The deformation amounts of the support sections also become more uniform, improving stability during insertion.

The sum of the refractive power along a line extending from the geometrical center to one outer circumference along a virtual cross-section and the refractive power along a line extending from the geometrical center to the other outer circumference along the virtual cross-section is calculated for each of virtual cross-sections defined by any direction. Then, the sum of the refractive powers on the lines along the second cross-section may be made equal to or less than the sum of the refractive powers on the lines along any other virtual cross-section.

As described above, the thickness at the lens side edge at a part (i.e., a part of the lens surface having a gentle curve) with a small refractive power is greater than that at a part (i.e., a part of the lens surface having a steep curve) with a large refractive power. Therefore, by designing the intraocular lens so that the sum in the second cross-section is equal to or greater than that in other directions, the thickness of the side of the lens section at the connection points of the support sections is increased.

The method for defining the direction of the second cross-section may also be changed. For example, the sum of the radius of curvature (e.g., an average value of the radii of curvature along the line) along a line extending from the geometrical center to one outer circumference along a virtual cross-section and the radius of curvature (e.g., an average value of the radii of curvature along the line) along a line extending from the geometrical center to the other outer circumference along the virtual cross-section is calculated for each of virtual cross-sections defined by any direction. Then, the sum of the radii of curvature along the pair of lines extending along the second cross-section may be made equal to or greater than the sum of the radii of curvature along the pair of lines extending along any other virtual cross-section. Even in this case, the thickness at the connection points of the support sections is increased.

In the toric intraocular lens, the position of the second cross-section may be made to coincide with the weak principal meridian. Even in this case, the thickness at the connection points of the support sections is increased.

At least one of the front and rear surfaces of the lens section may be formed with multiple segmental regions. The multiple segmental regions may radiate outwardly from the center and have mutually different refractive powers due to different radii of curvature. The segmental regions may be arranged so that the cross-sectional area in the first cross-section is equal to or less than that in other directions.

In this case, unlike intraocular lenses with multiple regions arranged concentrically, even if the wearer's pupil becomes small, light passes through each segmental region and is focused on the retina. The multiple segmental regions have mutually different refractive powers due to different radii of curvature. Therefore, compared to forming microprism groups or the like, the amount of light scattered in unintended directions is reduced. As a result, loss of light reaching the retina is less likely, making it easier to obtain a better visual field. Furthermore, the intraocular lens is more easily processed with high precision and less likely to be damaged during insertion. The intraocular lens is folded small in the passage of the nozzle and inserted into the eye.

However, as described above, the technology exemplified in the second aspect of the present disclosure can also be applied to intraocular lenses without multiple segmental regions (e.g., toric intraocular lenses). It is also possible for both multiple segmental regions and a toric surface for correcting astigmatism to be formed in the lens section. The surfaces on which the segmental regions and the toric surface are formed may be the same or different.

[Brief Description of Drawings]

[FIG. 1] FIG. 1 is a plan view of intraocular lens 1.

[FIG. 2] FIG. 2 is a perspective view of intraocular lens insertion instrument 100 from the upper right.

[FIG. 3] FIG. 3 is a perspective view of plunger 300 from the upper right

[FIG. 4] FIG. 4 is a schematic explanatory diagram showing the state where movement and deformation of intraocular lens 1 are appropriately performed.

[FIG. 5] FIG. 5 is a plan view showing an example of intraocular lens 1 with multiple segmental regions 20 formed.

[FIG. 6] FIG. 6 is a schematic comparison of cross-sectional views of the distance region 20A and near region 20B passing through the geometric center O of lens section 2 and perpendicular to the lens surface.

[FIG. 7] FIG. 7 is a comparison of MTF curves of four intraocular lenses 1 with different refractive powers and central angles for distance region 20A, near region 20B, and intermediate region 20C.

[FIG. 8] FIG. 8 is a comparison of MTF curves of three intraocular lenses with different refractive powers for distance region 20A, near region 20B, and intermediate region 20C.

[FIG. 9] FIG. 9 is a comparison of MTF curves of intraocular lens 1 with and without a transition region between the distance and near regions.

[FIG. 10] FIG. 10 is a diagram showing lines for calculating the sum of refractive powers in intraocular lens 1 shown in FIG. 5

[DESCRIPTION OF EMBODIMENTS]

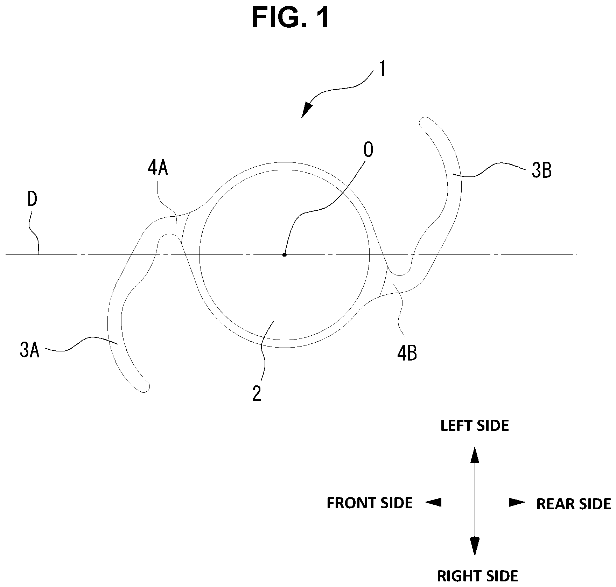

The following describes typical embodiments of the present disclosure with reference to the drawings. Intraocular lens 1 of this embodiment is inserted into the patient's eye using an intraocular lens insertion instrument 100. In the following description, the side of the intraocular lens 1 that is first inserted into the eye by the insertion instrument 100 (left side in FIG. 1) is referred to as the front of the intraocular lens 1.

(Outline Structure of Intraocular Lens)

With reference to FIG. 1, the outline structure of intraocular lens 1 of this embodiment is described. Intraocular lens 1 comprises a lens section 2 and a support section 3. Intraocular lens 1 of this embodiment is a so-called one-piece intraocular lens in which the lens section 2 and support section 3 are integrally molded. However, at least part of the technology exemplified in the present disclosure can also be applied to so-called three-piece intraocular lenses, in which lens section 2 and support section 3 are formed as separate members. Intraocular lens 1 is deformable. The material for intraocular lens 1 may be, for example, various soft materials such as BA (butyl acrylate), HEMA (hydroxyethyl methacrylate), or composite materials of acrylic and methacrylic acid esters.

The lens section 2 provides a predetermined refractive power to the wearer's eye (i.e., the patient's eye). Details of the refractive power imparted to lens section 2 are described later. The shape of lens section 2 is disc-shaped. The optical axis of lens section 2 exemplified in this embodiment passes through the geometric center O and extends in a direction perpendicular to the lens surface (vertical direction). However, the optical axis of lens section 2 does not necessarily have to coincide with the geometric center O.

The intraocular lens 1 of this embodiment comprises a pair of support sections 3 (a front support section 3A and a rear support section 3B). The base ends 4 of the pair of support sections 3 (i.e., base end 4A of front support section 3A and base end 4B of rear support section 3B) are connected to different parts of the lens edge (in this embodiment, diametrically opposite parts of the disc-shaped lens section 2). When intraocular lens 1 is implanted in the patient's eye, the pair of support sections 3 support lens section 2 inside the eye.

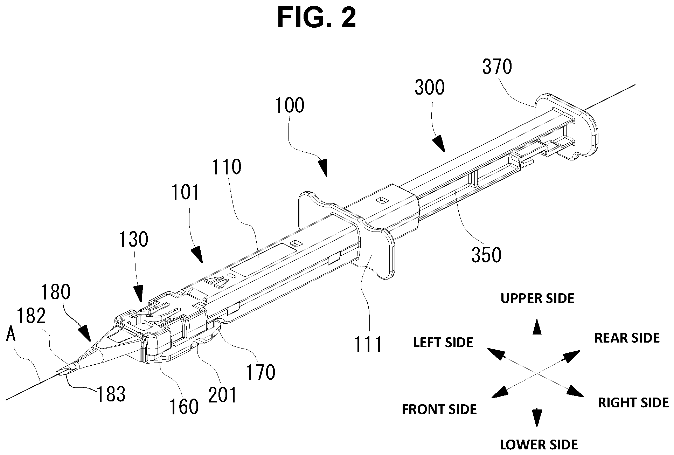

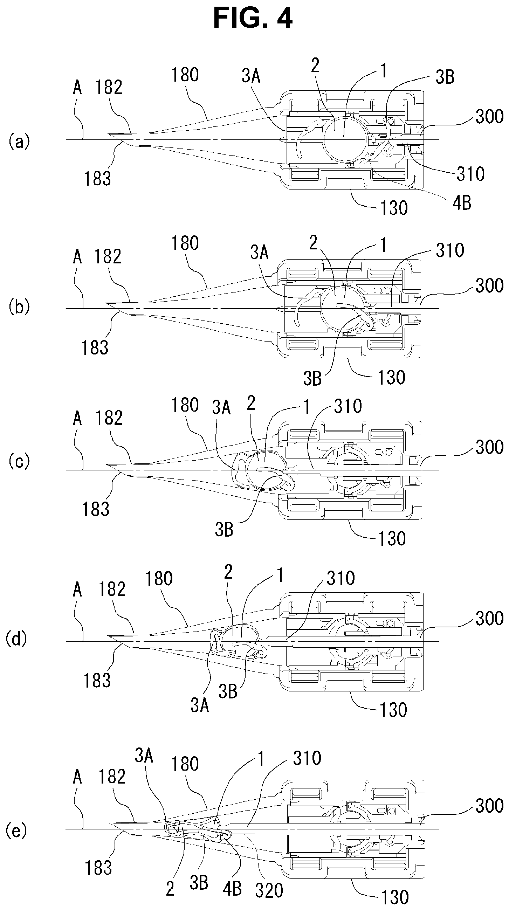

As described later, in this embodiment, the expected direction of movement D of the intraocular lens 1 as it travels through the passage of a nozzle 180 (see FIGS. 2 and 4) of the insertion instrument 100 is predetermined. That is, the expected direction of the movement D of the intraocular lens 1 as it travels through the passage of nozzle 180 coincides with the extrusion axis A (see FIGS. 2 and 4), which is the axis of the passage.

Front support section 3A extends curvedly forward from the lens edge when intraocular lens 1 is set in installation section 130 of insertion instrument 100 (see FIG. 4). That is, front support section 3A has a loop shape curved in the circumferential direction, and its tip is a free end. Rear support section 3B extends curvedly backward from the lens edge when intraocular lens 1 is set in installation section 130 of insertion instrument 100 (see FIG. 4). That is, rear support section 3B also has a loop shape curved in the circumferential direction, and its tip is a free end.

(Intraocular Lens Insertion Instrument)

The intraocular lens insertion instrument 100 is described. In the following description, the direction toward the nozzle 180 side of main body 101 (lower left in FIG. 2) is referred to as the front of insertion instrument 100, and the direction toward the pressing section 370 of plunger 300 (upper right in FIG. 2) is referred to as the rear. The upper side of FIG. 2 is the upper side of insertion instrument 100, the lower side is the lower side, the lower right is the right side, and the upper left is the left side.

With reference to FIG. 2, the overall structure of intraocular lens insertion instrument 100 of this embodiment is described. As described above, the insertion instrument 100 is used to insert the deformable intraocular lens 1 into the eye. The insertion instrument 100 comprises a main body 101 and a plunger 300. The main body 101 is substantially tubular, and intraocular lens 1 is inserted into the eye through its internal passage. The plunger 300 is rod-shaped and can move back and forth in the internal passage of the main body 101. The plunger 300 moves forward along extrusion axis A (the axis of the passage) to push intraocular lens 1 loaded in main body 101 forward.

The main body 101 and the plunger 300 of this embodiment are formed of resin material. The insertion instrument 100 may be formed by molding, cutting, or other processing of resin. Forming insertion instrument 100 from resin allows users to easily dispose of it after use.

In this embodiment, a lubricating coating is applied to the inner wall of main body 101 to facilitate smooth insertion of the soft, adhesive intraocular lens 1. The insertion instrument 100 is formed of colorless transparent or colorless semi-transparent material. Therefore, users can easily visually check the deformation state of intraocular lens 1 loaded inside insertion instrument 100 from the outside.

With reference to FIG. 2, the main body 101 is described. The main body 101 comprises a main tube section 110, an installation section 130, and a nozzle (an insertion section) 180.

The main tube section 110 is formed in a tubular shape extending in the front-rear direction and is located at the rear end side (base end side) of main body 101. A protruding section 111 for gripping by the user is formed slightly forward of the rear end of main tube section 110.

The installation section 130 is connected to the front end side of the main tube section 110. The intraocular lens 1 is installed (loaded) in installation section 130. Specifically, installation section 130 comprises holding section 160 and setting section 170. Holding section 160 holds intraocular lens 1 when insertion instrument 100 is in storage state. Setting section 170 is provided to be rotatable about the axis of its tip. When setting section 170 is rotated, intraocular lens 1 held in holding section 160 is moved to a standby position where it can be pushed out by plunger 300 and positioned.

The nozzle 180 is connected to the front end side of the installation section 130. The passage area inside nozzle 180 is formed to decrease toward the front to deform intraocular lens 1 into a smaller shape as it is pushed forward. That is, a tapered internal space is formed in nozzle 180. At the front end of nozzle 180, a cylindrical insertion section 182 with a diagonally cut tip is provided. Insertion section 182 is inserted into the eye. At the front end of insertion section 182, an opening 183 for discharging intraocular lens 1 from the internal passage to the front is formed. The internal passage of main body 101 runs from the rear end of main tube section 110 to the insertion opening 183 at the front end of nozzle 180.

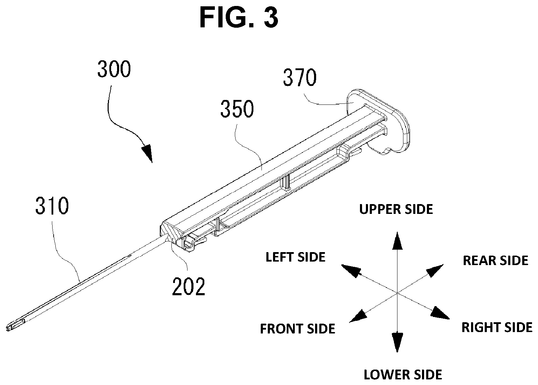

With reference to FIG. 3, the outline structure of the plunger 300 is described. Plunger 300 of this embodiment comprises an extrusion member 310, a shaft base 350, and a pressing section 370.

The pressing section 370 is formed at the rear end of the plunger 300. Pressing section 370 is a plate-shaped member extending in a direction perpendicular to extrusion axis A (see FIG. 2). The user's finger contacts pressing section 370 when pushing plunger 300 forward.

The shaft base 350 is a rod-shaped member extending forward from the front end of the pressing section 370. In this embodiment, shaft base 350 is formed so that its cross-section perpendicular to extrusion axis A is substantially H-shaped. By inserting shaft base 350 into main tube section 110, whose cross-section perpendicular to extrusion axis A is substantially rectangular, rotation of plunger 300 about extrusion axis A relative to main body 101 is suppressed. When plunger 300 moves forward and reaches the position where insertion of intraocular lens 1 into the eye is complete, the inclined surface at the lower front end of shaft base 350 contacts the inclined surface formed at a predetermined location in main body 101. As a result, excessive protrusion of the front end of plunger 300 from insertion opening 183 (see FIG. 2) is prevented.

The extrusion member 310 is a rod-shaped member extending forward from the front end of shaft base 350 along extrusion axis A. Extrusion member 310 is formed so that its cross-section perpendicular to extrusion axis A is substantially circular. The thickness of extrusion member 310 is such that it can pass through insertion opening 183 of main body 101. Extrusion member 310 moves forward along extrusion axis A in the internal passage of main body 101, pushing intraocular lens 1 forward while folding it small and discharging it from insertion opening 183 into the eye.

With reference to FIG. 4, the movement and deformation of intraocular lens 1 when inserted into the eye using insertion instrument 10 of this embodiment are described. First, the operator rotates setting section 170 (see FIG. 2) to move intraocular lens 1 held in holding section 160 (see FIG. 2) to a standby position where it can be pushed out by plunger 300. As shown in FIG. 4(a), the base end 4B of rear support section 3B in intraocular lens 1 held in holding section 160 is offset to either the left or right relative to extrusion axis A.

Next, the operator injects a lubricant (viscoelastic substance) into installation section 130 using an injector or the like and starts moving plunger 300 forward. As a result, as shown in FIG. 4(a), extrusion member 310 contacts rear support section 3B of intraocular lens 1.

As plunger 300 is pushed further forward, as shown in FIG. 4(b), the rear support section 3B moves (is bent) toward lens section 2 by extrusion member 310. As a result, rear support section 3B is deformed and moved above lens section 2, and its tip faces forward. The state shown in FIG. 4(b) may be referred to as rear support section 3B being tacked.

As plunger 300 is pushed further forward, the extrusion member 310 contacts lens section 2, and the entire intraocular lens 1 moves forward. When intraocular lens 1 reaches nozzle 180, as shown in FIG. 4(c), front support section 3A contacts the inner wall of the tapered nozzle 180. As a result, front support section 3A is deformed and moved above lens section 2, and its tip faces backward. That is, front support section 3A is tacked. Also, by front support section 3A contacting the inner wall of the tapered nozzle 180, the expected direction of movement D (see FIG. 1) of intraocular lens 1 coincides with extrusion axis A of insertion instrument 100.

As the plunger 300 is pushed further forward, lens section 2 of intraocular lens 1 also contacts the inner wall of the tapered nozzle 180. As a result, as shown in FIG. 4(d), lens section 2 is folded small.

As shown in FIG. 4(e), the intraocular lens 1 is folded smaller as it is pushed forward. Since the passage area of nozzle 180 becomes narrower toward the front, the load on intraocular lens 1 may gradually increase. However, intraocular lens 1 of this embodiment is designed to be more easily folded small by nozzle 180. Details are described later.

(Segmental Regions of the Lens Section)

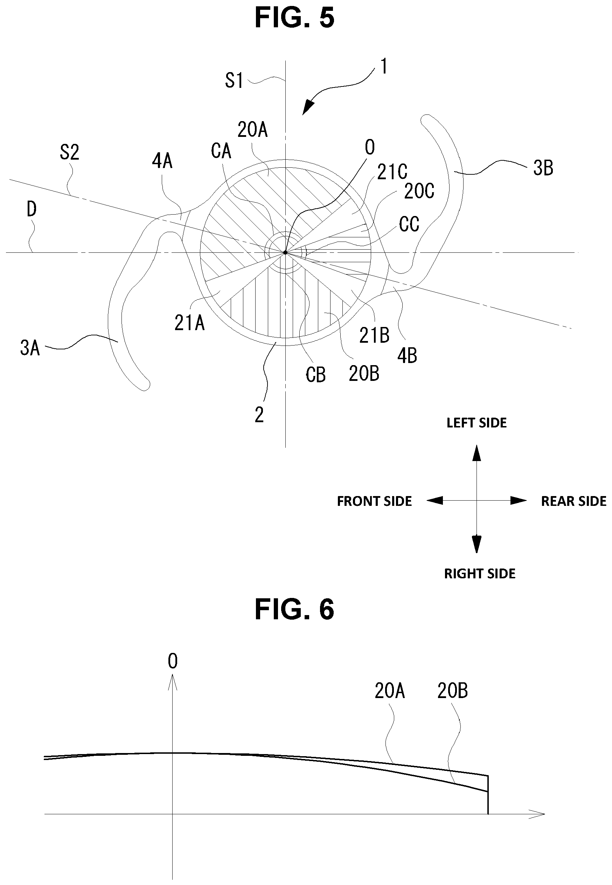

The segmental regions 20 formed in the lens section 2 of the intraocular lens 1 of this embodiment are described. As shown in FIG. 5, in the intraocular lens 1 of this embodiment, at least one of the front and rear surfaces of the disc-shaped lens section 2 is formed with three or more segmental regions 20 (20A, 20B, 20C).

The multiple segmental regions 20 have mutually different refractive powers. Specifically, lens section 2 of this embodiment is formed with distance region 20A, near region 20B, and intermediate region 20C. Distance region 20A has the smallest refractive power among the segmental regions 20. Near region 20B has the largest refractive power among the segmental regions 20. Intermediate region 20C has a refractive power between those of distance region 20A and near region 20B. Thus, light passing through distance region 20A, near region 20B, and intermediate region 20C in lens section 2 is focused on the wearer's retina, achieving the multifocal effect. In the present disclosure, each segmental region 20 is a region in which the refractive power is substantially uniform regardless of the location within the region.

When lens section 2 is viewed from a direction perpendicular to the lens surface, the multiple segmental regions 20 radiate outward from the center of lens section 2. That is, each segmental region 20 is segmented by a boundary line extending straight from a single point at the center of lens section 2 outward. Therefore, unlike intraocular lenses with multiple regions arranged concentrically, even if the wearer's pupil becomes small, light can easily pass through all of the multiple segmental regions 20. As a result, the multifocal effect is more easily achieved regardless of pupil size. The boundary lines of segmental regions 20 are not limited to straight lines and may be curved or bent.

Specifically, in intraocular lens 1 of this embodiment, the segmental regions 20 radiate outward from a single reference point at the center (in this embodiment, the geometric center O) of lens section 2. That is, the straight boundary lines (ends) of each segmental region 20 extending from the center of lens section 2 outward all pass through the same reference point. In other words, the segmental regions 20 are arranged in a circumferential direction around the center of the lens section 2. Therefore, even if the wearer's pupil becomes small, the amount of light passing through each segmental region 20 is more easily ensured compared to the case where a certain region (e.g., a circular region) is separately formed at the center of lens section 2. As a result, the multifocal effect is more easily achieved regardless of pupil size, making it easier for the wearer to have a good visual field. However, it is also possible to provide a certain region at the center of lens section 2. Even in this case, the multifocal effect is more easily achieved regardless of pupil size compared to the case where the regions are arranged concentrically.

FIG. 6 is a schematic comparison of cross-sectional views of the distance region 20A and the near region 20B passing through the geometric center O of lens section 2 and perpendicular to the lens surface. As shown in FIG. 6, the multiple segmental regions 20 formed in intraocular lens 1 of this embodiment have mutually different refractive powers due to different radii of curvature. The lens surfaces of each segmental region 20 may be spherical or aspherical. If the lens surface is aspherical, the term "radius of curvature" in the present disclosure may be interpreted as the radius of curvature of a sphere approximating the aspherical lens surface.

In the example shown in FIG. 6, the radius of curvature of distance region 20A with a small refractive power is larger than that of near region 20B with a large refractive power. In other words, the curvature of the lens surface in distance region 20A with a small refractive power is gentler than that in near region 20B with a large refractive power. By varying the radii of curvature of each segmental region 20 to change their refractive powers, the amount of light scattered in unintended directions is reduced compared to forming microprism groups or the like in the segmental regions. As a result, loss of light reaching the retina is less likely, making it easier to obtain a better visual field. Furthermore, intraocular lens 1 of this embodiment is easier to process with high precision and less likely to be damaged during insertion.

As shown in FIG. 6, no step or the like is provided at the geometric center O of lens section 2. Therefore, the thickness at the lens edge at a part with a large refractive power (i.e., a part of the lens surface having a steep curve) is smaller than that at a part with a small refractive power (i.e., a part of the lens surface having a gentle curve).

As shown in FIG. 5, when lens section 2 is viewed from a direction perpendicular to the lens surface, a transition region 21 (21A, 21B, 21C) is formed between the multiple segmental regions 20 in lens section 2. Each transition region 21 smoothly connects the ends of a pair of adjacent segmental regions 20 by continuously changing the radius of curvature from the end of one segmental region 20 to the end of the other.

In the example shown in FIG. 5, the refractive power of distance region 20A is 0 D, that of near region 20B is +3.25 D, and that of intermediate region 20C is +2.0 D. Therefore, in transition region 21A formed between distance region 20A and near region 20B, the refractive power smoothly changes from 0 D at the end of distance region 20A to +3.25 D at the end of near region 20B. In transition region 21B formed between near region 20B and intermediate region 20C, the refractive power smoothly changes from +3.25 D at the end of near region 20B to +2.0 D at the end of intermediate region 20C. In transition region 21C formed between intermediate region 20C and distance region 20A, the refractive power smoothly changes from +2.0 D at the end of intermediate region 20C to 0 D at the end of distance region 20A.

If no transition region 21 is formed in the lens section 2, steps or the like may occur at the boundaries of adjacent segmental regions 20, which can cause light to be diffusely reflected and degrade the visual field (e.g., halo or glare). In contrast, forming a transition region 21 between adjacent segmental regions 20 reduces the influence of such diffuse reflection. Furthermore, when a transition region 21 is formed, the refractive power in transition region 21 smoothly transitions from the refractive power of one adjacent segmental region 20 to that of the other. As a result, in addition to the refractive powers of each segmental region 20, the lens section is also provided with refractive powers between those of the segmental regions. Thus, forming a transition region 21 in the lens section appropriately provides the effect of extended depth of focus (EDOF) in multifocal intraocular lenses.

In intraocular lens 1 of this embodiment, both the segmental regions 20 and transition regions 21 refract light entering lens section 2 parallel to the optical axis toward the optical axis. As a result, the amount of light passing through lens section 2 but not reaching the retina is reduced, making it easier to obtain a better visual field.

The central angle of each transition region 21 radiating outward from the center (reference point) of lens section 2 is designed to be 5 degrees or more and 30 degrees or less. In this case, both the multifocal effect and the extended depth of focus effect from the multiple segmental regions 20 are more easily achieved. More preferably, the central angle of each transition region 21 is designed to be 15 degrees or more and 20 degrees or less. In the example shown in FIG. 5, the central angle of each of the three transition regions 21 formed in intraocular lens 1 is designed to be 20 degrees. However, when multiple transition regions 21 are formed in lens section 2, their central angles need not be the same.

The central angles of distance region 20A, near region 20B, and intermediate region 20C radiating outward from the center of lens section 2 are designed so that the central angle CA of distance region 20A is the largest and the central angle CC of intermediate region 20C is the smallest. In this case, both distance vision by distance region 20A and near vision by near region 20B are more easily obtained with extended depth of focus.

Specifically, the central angle CA of distance region 20A is designed to be 140 degrees or more, the central angle CB of near region 20B is designed to be 90 degrees or more, and the central angle CC of intermediate region 20C is designed to be 30 degrees or more. In this case, both distance vision by distance region 20A and near vision by near region 20B are more easily obtained with extended depth of focus. In the example shown in FIG. 5, the central angle CA of distance region 20A is 150 degrees, the central angle CB of near region 20B is 100 degrees, and the central angle CC of intermediate region 20C is 50 degrees.

In the intraocular lens 1 of this embodiment, the refractive power of near region 20B is designed to be +2.5 D or more and +4.0 D or less, and the refractive power of intermediate region 20C is designed to be +1.0 D or more and +2.5 D or less. In this case, both the multifocal effect and the extended depth of focus effect from the multiple segmental regions 20 are more easily achieved. More preferably, the refractive power of near region 20B is designed to be +3.0 D or more and +3.5 D or less, and the refractive power of intermediate region 20C is designed to be +1.5 D or more and +2.0 D or less. In the example shown in FIG. 5, the refractive power of distance region 20A is 0 D, that of near region 20B is +3.25 D, and that of intermediate region 20C is +2.0 D.

At least one of the front and rear surfaces of lens section 2 may be formed with a toric surface to correct the wearer's astigmatism. In this case, an intraocular lens 1 is provided that not only enables both distance and near vision but also corrects the wearer's astigmatism. The multiple segmental regions 20 may be formed on the front surface of the lens (i.e., the surface facing the front of the eye (corneal side) when implanted). In this case, the rear surface of the lens is more likely to be smooth, suppressing the entry of cells between the rear surface and the posterior capsule of the eye, thereby reducing the occurrence of posterior capsule opacification. It is also possible for both the multiple segmental regions 20 and the toric surface to be formed on the front surface. In this case, even if a toric surface is formed, posterior capsule opacification is more easily suppressed. However, it is also possible for at least one of the segmental regions 20 and the toric surface to be formed on the rear surface. The surfaces on which the segmental regions 20 and the toric surface are formed may be the same or different.

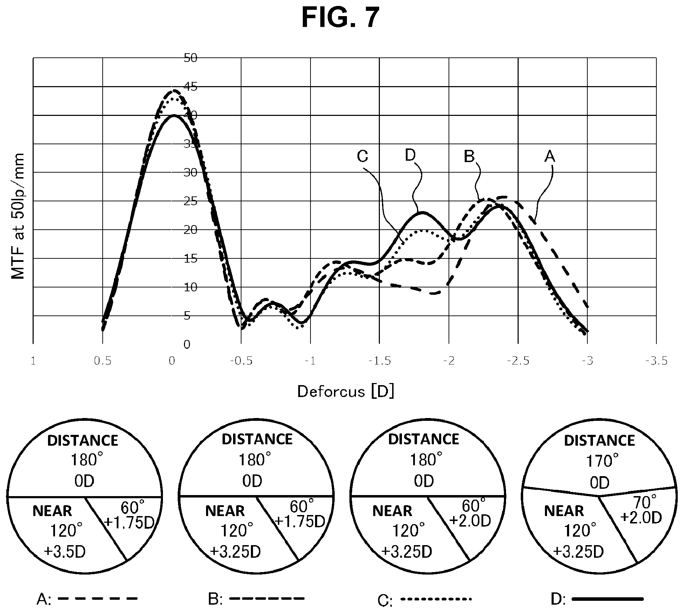

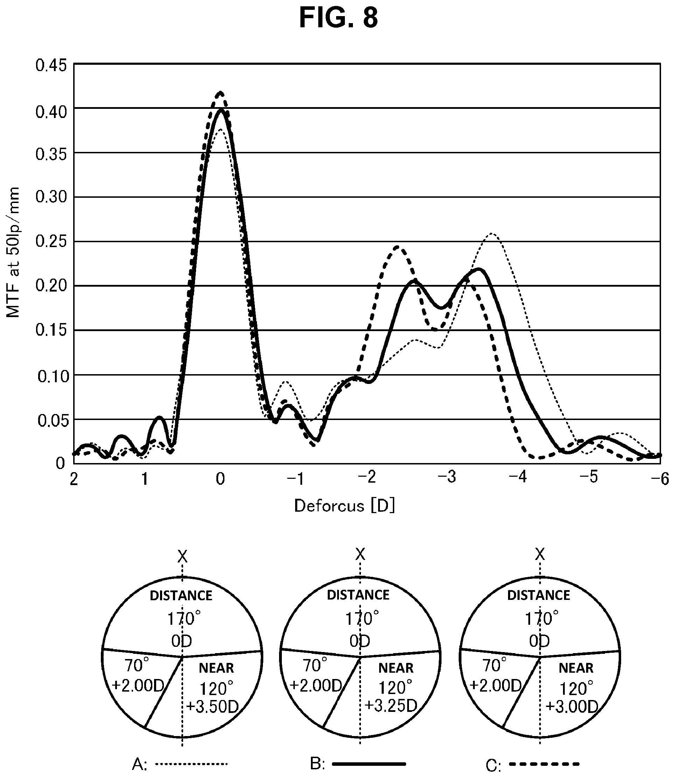

With reference to FIGS. 7 and 8, the MTF characteristics when at least one of the refractive power and central angle of each of the distance region 20A, near region 20B, and intermediate region 20C is changed will be described. MTF (Modulation Transfer Function) is an index indicating contrast. The graphs in FIGS. 7 and 8 show MTF curves at a spatial frequency of 50 lp/mm, with defocus amount (focal shift, power shift amount) on the horizontal axis and MTF on the vertical axis. In this embodiment, the MTF curves shown represent the case where the analysis direction of light intensity passed through the lens section 2 of intraocular lens 1 to obtain MTF is parallel to line X that bisects the central angle of distance region 20A.

In the four intraocular lenses 1 (A, B, C, D) whose MTF curves are shown in FIG. 7, the refractive powers and central angles of distance region 20A, near region 20B, and intermediate region 20C are changed within the ranges of the desirable conditions mentioned above. Specifically, in intraocular lens A, the central angle of distance region 20A is 180 degrees with refractive power 0 D, the central angle of near region 20B is 120 degrees with refractive power +3.5 D, and the central angle of intermediate region 20C is 60 degrees with refractive power +1.75 D. In intraocular lens B, the central angle of distance region 20A is 180 degrees with refractive power 0 D, the central angle of near region 20B is 120 degrees with refractive power +3.25 D, and the central angle of intermediate region 20C is 60 degrees with refractive power +1.75 D. In intraocular lens C, the central angle of distance region 20A is 180 degrees with refractive power 0 D, the central angle of near region 20B is 120 degrees with refractive power +3.25 D, and the central angle of intermediate region 20C is 60 degrees with refractive power +2.0 D. In intraocular lens D, the central angle of distance region 20A is 170 degrees with refractive power 0 D, the central angle of near region 20B is 120 degrees with refractive power +3.25 D, and the central angle of intermediate region 20C is 70 degrees with refractive power +2.0 D. In the four intraocular lenses 1 (A, B, C, D) whose MTF curves are shown in FIG. 7, transition regions with central angles of 5 degrees are actually provided between each region.

As shown in FIG. 7, in all four intraocular lenses 1 (A, B, C, D), MTF has a maximum value near defocus amount 0 D corresponding to distance vision, and also has maximum values in the range of defocus amount -1.5 D to -2.5 D corresponding to near vision. Furthermore, in each intraocular lens 1, the depth of focus is appropriately extended for both distance vision and near vision. From these results, it can be seen that by setting the refractive powers and central angles of distance region 20A, near region 20B, and intermediate region 20C within the ranges of the desirable conditions mentioned above, distance vision and near vision can both be appropriately obtained with extended depth of focus. It can also be seen that the MTF characteristics of intraocular lens 1 can be adjusted by appropriately adjusting the refractive powers and central angles of distance region 20A, near region 20B, and intermediate region 20C.

In the three intraocular lenses 1 (A, B, C) whose MTF curves are shown in FIG. 8, the central angles of distance region 20A, near region 20B, and intermediate region 20C are made identical, and only the refractive powers of each region are changed. Specifically, in intraocular lens A, the central angle of distance region 20A is 170 degrees with refractive power 0 D, the central angle of near region 20B is 120 degrees with refractive power +3.50 D, and the central angle of intermediate region 20C is 70 degrees with refractive power +2.00 D. As will be described in detail later, intraocular lens A is merely shown as a comparative example. In intraocular lens B, the central angle of distance region 20A is 170 degrees with refractive power 0 D, the central angle of near region 20B is 120 degrees with refractive power +3.25 D, and the central angle of intermediate region 20C is 70 degrees with refractive power +2.00 D. In intraocular lens C, the central angle of distance region 20A is 170 degrees with refractive power 0 D, the central angle of near region 20B is 120 degrees with refractive power +3.00 D, and the central angle of intermediate region 20C is 70 degrees with refractive power +2.0 D. In the three intraocular lenses 1 (A, B, C) whose MTF curves are shown in FIG. 8, transition regions with central angles of 20 degrees are actually provided between each region. Therefore, in the three intraocular lenses 1 (A, B, C), excluding the angles of the transition regions, the central angle of distance region 20A is 150 degrees, the central angle of near region 20B is 100 degrees, and the central angle of intermediate region 20C is 50 degrees.

In intraocular lenses B and C, the refractive powers of each region are designed so that three maximum values (distance maximum, near maximum, and intermediate maximum) with MTF values of 0.10 or more appear in the MTF curve at spatial frequency 50 lp/mm. The distance maximum is the maximum value in the defocus amount range that provides distance vision to the wearer (in this embodiment, the range of defocus amount -1.0 D to 1.0 D). The near maximum is the maximum value in the defocus amount range that provides near vision to the wearer (in this embodiment, the range where defocus amount is more negative than the distance maximum). The intermediate maximum is the maximum value in the range where defocus amount is between the distance maximum and near maximum. The MTF curves illustrated in FIG. 8 are MTF curves based on the cornea, where the negative value increases as the distance of vision provided to the wearer approaches near. However, in MTF curves based on the surface of intraocular lens 1, the positive value increases as the distance of vision provided to the wearer approaches near.

As described above, in the intraocular lens 1 of this embodiment, multiple segmental regions with mutually different refractive powers (distance region 20A, near region 20B, and intermediate region 20C) radiate outwardly from the center of the lens section. Therefore, unlike intraocular lenses with multiple regions arranged concentrically, even when the wearer's pupil becomes small, light easily passes through all of the multiple segmental regions. Furthermore, by designing intraocular lenses 1 like those exemplified in B and C so that three maximum values (distance maximum, near maximum, and intermediate maximum) with MTF values of 0.10 or more appear, unlike comparative example A, good vision is easily obtained not only at distance and near distances but also at intermediate distances between distance and near. In other words, according to intraocular lenses 1 exemplified in B and C, regardless of pupil size, good vision at distance, near, and intermediate distances is easily obtained.

As shown in FIG. 8, in the intraocular lenses B and C, the MTF curve at spatial frequency 50 lp/mm is designed so that the difference between the smaller MTF value of the near maximum and intermediate maximum and the minimum value existing between the near maximum and intermediate maximum is 0.10 or less. Specifically, in intraocular lens B, the difference between the smaller MTF value (intermediate maximum in intraocular lens B) of approximately 0.21 and the minimum value existing between the near maximum and intermediate maximum (approximately 0.18) is approximately 0.03 (≤0.10). In intraocular lens C, the difference between the smaller MTF value (near maximum in intraocular lens C) of approximately 0.21 and the minimum value existing between the near maximum and intermediate maximum (approximately 0.15) is approximately 0.06 (≤0.10). In these cases, distances with reduced vision are less likely to be included between near and intermediate distances. In other words, good vision is easily obtained overall from near to intermediate distances. Therefore, regardless of pupil size, vision at distance, near, and intermediate distances is even more easily obtained.

Specifically, in the intraocular lenses B and C, the MTF curve at spatial frequency 50 lp/mm is designed so that the difference between the smaller MTF value of the near maximum and intermediate maximum and the minimum value existing between the near maximum and intermediate maximum is 0.08 or less. In this case, vision from near to intermediate distances is even more easily obtained.

More specifically, in the intraocular lens B, the MTF curve at spatial frequency 50 lp/mm is designed so that the difference between the smaller MTF value of the near maximum and intermediate maximum and the minimum value existing between the near maximum and intermediate maximum is 0.05 or less. In this case, vision from near to intermediate distances is even more easily obtained.

As shown in FIG. 8, in intraocular lens B, the refractive powers of each region are designed so that the near maximum is greater than the intermediate maximum in the MTF curve at spatial frequency 50 lp/mm. By satisfying the condition that the near maximum is greater than the intermediate maximum, contrast at frequently viewed near distances becomes high. As a result, better vision is more easily obtained.

As shown in FIG. 8, in intraocular lenses B and C, the refractive powers of each region are designed so that the difference between the near maximum and intermediate maximum is 0.05 or less in the MTF curve at spatial frequency 50 lp/mm. Specifically, in intraocular lens B, the difference between the near maximum and intermediate maximum is approximately 0.02 ($\leq$0.05). In intraocular lens C, the difference between the near maximum and intermediate maximum is approximately 0.03 ($\leq$0.05). In these cases, good vision is easily obtained at both near and intermediate distances. Therefore, regardless of pupil size, vision at distance, near, and intermediate distances is even more easily obtained.

As a result of repeated trial and error and simulation by the inventors of the present invention, it was newly discovered that by bringing the difference between the refractive power S of near region 20B and the refractive power M of intermediate region 20C to 1.4 D or less, compared to making the difference greater than 1.4 D, a synergistic effect is obtained where the intermediate maximum in the MTF curve increases and MTF values from intermediate to near distances increase overall.

Actually, as shown in FIG. 8, in comparative example A, the difference between the refractive power S of near region 20B and the refractive power M of intermediate region 20C is 1.5 D (>1.4 D). In intraocular lens A, MTF values at intermediate distances are insufficient, and MTF values from intermediate to near distances are also insufficient overall. In contrast, in intraocular lenses B and C, the difference between the refractive power S of near region 20B and the refractive power M of intermediate region 20C is smaller than 1.4 D. Specifically, in intraocular lens B, the difference between the refractive power S of near region 20B and the refractive power M of intermediate region 20C is 1.25 D ($\leq$1.4 D). In intraocular lens C, the difference between the refractive power S of near region 20B and the refractive power M of intermediate region 20C is 1.00 D ($\leq$1.4 D). In intraocular lenses B and C, compared to intraocular lens A, it can be seen that a synergistic effect occurs where the intermediate maximum in the MTF curve increases and MTF values from intermediate to near distances increase overall. In other words, in intraocular lenses B and C, when the refractive power of the near region is S and that of the intermediate region is M, by satisfying both conditions $S \geq +2.75$ D and $M < S \leq (M + 1.4$ D), good vision at distance, near, and intermediate distances is easily obtained regardless of pupil size.

Intraocular lenses B and C may satisfy both conditions $S \geq +2.75$ D and $M < S \leq (M + 1.35$ D). That is, the difference between the refractive power S of the near region and the refractive power M of the intermediate region may be 1.35 D or less. In this case, the effect of increasing the intermediate maximum in the MTF curve and the effect of overall increase in MTF values from intermediate to near distances are even more easily obtained.

In the intraocular lens B, when the refractive power of the near region is S and that of the intermediate region is M, both conditions $S \geq +2.75$ D and $(M + 1.1$ D$) \leq S \leq (M + 1.4$ D$)$ are satisfied. As mentioned above, a useful synergistic effect is obtained by bringing the difference between the refractive power S of the near region and the refractive power M of the intermediate region to 1.4 D or less. On the other hand, if the difference between the refractive power S of the near region and the refractive power M of the intermediate region is made too small, the effect of multifocal intraocular lenses for obtaining good vision at distance, near, and intermediate distances decreases. Therefore, by designing intraocular lens 1 to satisfy both conditions $S \geq +2.75$ D and $(M + 1.1$ D$) \leq S \leq (M + 1.4$ D$)$, vision at distance, near, and intermediate distances is even more easily obtained through multifocal effects regardless of pupil size. As mentioned above, the value "1.4 D" in the above conditions may be "1.35 D".

The difference between the refractive power S of the near region and the refractive power M of the intermediate region may be 1.15 D or more. In this case, vision at distance, near, and intermediate distances is even more easily obtained through multifocal effects.

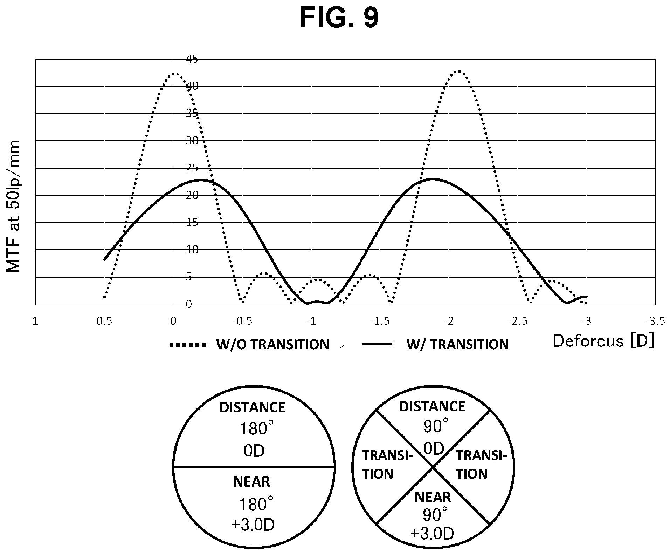

With reference to FIG. 9, the MTF characteristics when transition regions are not formed between distance and near regions versus when transition regions are formed will be described. The graph in FIG. 9 shows MTF curves at spatial frequency 50 lp/mm with defocus amount (focal shift, power shift amount) on the horizontal axis and MTF on the vertical axis, similar to the graphs in FIGS. 7 and 8.

FIG. 9 shows the MTF curve of an intraocular lens "without transition region" where no transition region is formed between distance and near regions, and the MTF curve "with transition region" where a transition region is formed between distance and near regions. Specifically, in the intraocular lens "without transition region," the central angle of the distance region is 180 degrees with refractive power 0 D, and the central angle of the near region is 180 degrees with refractive power +3.0 D. In the intraocular lens "with transition region," the central angle of the distance region is 90 degrees with refractive power 0 D, and the central angle of the near region is 90 degrees with refractive power +3.0 D. In the intraocular lens "with transition region," two transition regions with central angles of 90 degrees are formed between the distance and near regions.

As shown in FIG. 9, in the intraocular lens "with transition region," both the MTF maximum value corresponding to distance vision and the MTF maximum value corresponding to near vision are closer to each other compared to the intraocular lens "without transition region." Also, in the intraocular lens "with transition region," the depth of focus extension effect is improved compared to the intraocular lens "without transition region." As described above, it can be seen that by forming transition regions in the lens section of intraocular lenses, the extended depth of focus (EDOF) effect of multifocal intraocular lenses is appropriately obtained.

(Cross-sectional Area of Lens Section)

The cross-sectional area of lens section 2 of intraocular lens 1 of this embodiment will be described. As described with reference to FIG. 4, the intraocular lens of this embodiment is inserted into the patient's eye using intraocular lens insertion instrument 100. Specifically, intraocular lens 1 is moved forward along extrusion axis A in the passage of intraocular lens insertion instrument 100 by extrusion member 310. When intraocular lens 1 reaches nozzle 180, the expected movement direction D of intraocular lens 1 (see FIGS. 1 and 4) substantially coincides with extrusion axis A of intraocular lens insertion instrument 100 when front support section 3A contacts the inner wall of tapered nozzle 180 (i.e., the passage area becomes smaller toward the front). Subsequently, lens section 2 of intraocular lens 1 is folded small by contacting the inner wall of tapered nozzle 180.

As shown in FIG. 5, virtual cross-sections in any direction that pass through geometric center O of the lens section 2 and are perpendicular to the lens surface of the lens section 2 are assumed. A first cross-section S1 and a second cross-section S2 shown in FIG. 5 are examples of multiple assumed virtual cross-sections. As mentioned above, in the intraocular lens 1 of this embodiment, the cross-sectional area of the lens section 2 in the virtual cross-section changes depending on the direction along which the virtual cross-section is cut.

When the intraocular lens 1 is folded by the nozzle 180 of the intraocular lens insertion instrument 100, the virtual cross-section perpendicular to the extrusion axis A (see FIGS. 2 and 4) of intraocular lens insertion instrument 100 is designated as the first cross-section S1. As mentioned above, when the intraocular lens 1 reaches the nozzle 180, the expected movement direction D of the intraocular lens 1 coincides with the extrusion axis A. Therefore, the first cross-section S1 can also be expressed as a virtual cross-section perpendicular to the expected movement direction D of intraocular lens 1. In intraocular lens 1 of this embodiment, the cross-sectional area of lens section 2 in first cross-section S1 is equal to or less than the cross-sectional areas in virtual cross-sections in other directions. Therefore, when intraocular lens 1 is pushed through the passage of nozzle 180 at an appropriate angle to extrusion axis A, the maximum cross-sectional area of lens section 2 in the direction perpendicular to extrusion axis A (i.e., the cross-sectional area in first cross-section S1 where the cross-sectional area of folded lens section 2 is maximum) becomes small. Therefore, intraocular lens 1 of this embodiment can be folded small in the passage of nozzle 180.

The relationship between the first cross-section S1 and the configuration of lens section 2 will be further described. As described with reference to FIG. 6, in the lens section 2, the radius of curvature at parts with large refractive power is smaller than the radius of curvature at parts with small refractive power. In other words, in lens section 2, the curve of the lens surface at parts with large refractive power is steeper than the curve of the lens surface at parts with small refractive power. Since it is optically undesirable to provide steps at geometric center O of lens section 2, the thickness of the lens section at geometric center O in the virtual cross-section is the same regardless of the direction of the assumed virtual cross-section (see FIG. 6). As a result, the thickness of the side surface (edge) of lens section 2 at parts with large refractive power (parts where the lens surface curve is steep) becomes smaller than the thickness of the side surface of lens section 2 at parts with small refractive power (parts where the lens surface curve is gentle). That is, in lens section 2, the greater the refractive power at a part, the smaller the thickness.

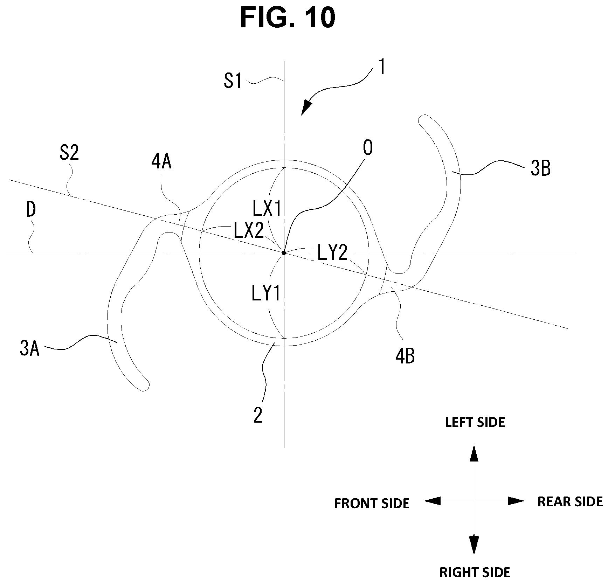

Here, in lens section 2, the sum of the refractive power along a line extending from geometric center O along the virtual cross-section to one periphery and the refractive power along a line extending from geometric center O along the same virtual plane to the other periphery is calculated for virtual cross-sections in each arbitrary direction. For example, in the example shown in FIG. 10, the sum of the refractive power of line LX1 extending from geometric center O along first cross-section S1 to one periphery (refractive power 0 D of distance region 20A shown in FIG. 5) and the refractive power of line LY1 extending from geometric center O along first cross-section S1 to the other periphery (refractive power +3.25 D of near region 20B shown in FIG. 5) is +3.25 D.

In intraocular lens 1 of this embodiment, lens section 2 is designed so that the sum of refractive powers along two lines LX1 and LY1 along first cross-section S1 is equal to or greater than the sum of refractive powers along two lines along virtual cross-sections in other directions. Therefore, since the thickness becomes smaller as the refractive power increases in lens section 2, the cross-sectional area of lens section 2 in first cross-section S1 becomes equal to or less than the cross-sectional area of lens section 2 in virtual cross-sections in other directions. As a result, the cross-sectional area of lens section 2 in the first cross-section S1 becomes appropriately small, so the intraocular lens 1 is easily folded small in the passage of nozzle 180.

The method of defining the configuration of lens section 2 in first cross-section S1 may be changed. For example, the sum of the radius of curvature of lens section 2 along a line extending from geometric center O along the virtual cross-section to one periphery (such as the average value of radii of curvature along the line; the same applies below) and the radius of curvature along a line extending from geometric center O along the same virtual cross-section to the other periphery is calculated for virtual cross-sections in each arbitrary direction. In this case, intraocular lens 1 may be designed so that the sum of radii of curvature along two lines LX1 and LY1 along first cross-section S1 is equal to or less than the sum of radii of curvature along two lines along virtual cross-sections in other directions. Even in this case, the cross-sectional area of lens section 2 in first cross-section S1 becomes appropriately small.

The relationship between base ends 4 (4A, 4B) of support sections 3 (3A, 3B) and lens section 2 will be described. As shown in FIGS. 1, 5, and 10, intraocular lens 1 of this embodiment includes a pair of support sections 3 (3A, 3B). The pair of support sections 3 extend outward from different parts of the side surface (edge) of lens section 2. It is desirable that base end 4 of each of the pair of support sections 3 be connected to a part with large thickness on the side surface of lens section 2 for stable support of lens section 2 in the eye. Also, if base end 4 of support section 3 is connected to a part with small thickness on the side surface of lens section 2, unintended deformation or damage is likely to occur in lens section 2 when support section 3 is bent during insertion of intraocular lens 1 into the eye.

Here, as shown in FIGS. 5 and 10, the virtual cross-section in the direction passing through base end 4 of each of the pair of support sections 3 is designated as second cross-section S2. In intraocular lens 1 of this embodiment, the cross-sectional area of lens section 2 in the second cross-section is equal to or greater than the cross-sectional areas in virtual cross-sections in other directions. As a result, the thickness of the parts where base ends 4 of the pair of support sections 3 are connected on the side surface of lens section 2 becomes large. Therefore, rigidity of base ends 4 of support sections 3 is ensured, so unintended deformation or damage is less likely to occur, and intraocular lens 1 is more easily positioned appropriately in the eye. Also, the deformation amounts of the pair of support sections 3 become more uniform, so stability during insertion of intraocular lens 1 into the eye is also easily improved.

The relationship between second cross-section S2 and the configuration of lens section 2 will be further described. In the example shown in FIG. 10, the sum of the refractive power of line LX2 extending from geometric center O along second cross-section S2 to one periphery (refractive power 0 D of distance region 20A shown in FIG. 5) and the refractive power of line LY2 extending from geometric center O along second cross-section S2 to the other periphery (refractive power +2.0 D of intermediate region 20C shown in FIG. 5) is +2.0 D. In intraocular lens 1 of this embodiment, lens section 2 is designed so that the sum of refractive powers along two lines LX2 and LY2 (see FIG. 10) along second cross-section S2 is equal to or less than the sum of refractive powers along two lines along virtual cross-sections in other directions. Therefore, since the thickness becomes larger as the refractive power decreases in lens section 2, the cross-sectional area of lens section 2 in second cross-section S2 becomes equal to or greater than the cross-sectional area of lens section 2 in virtual cross-sections in other directions. As a result, the thickness of the parts where base ends 4 of the pair of support sections 3 are connected on the side surface of lens section 2 becomes large.

The method of defining the configuration of lens section 2 in second cross-section S2 may be changed. For example, intraocular lens 1 may be designed so that the sum of radii of curvature along two lines LX2 and LY2 along second cross-section S2 is equal to or greater than the sum of radii of curvature along two lines along virtual cross-sections in other directions. Even in this case, the thickness of the parts where base ends 4 of the pair of support sections 3 are connected on the side surface of lens section 2 becomes large.

As shown in FIG. 5, in lens section 2 of intraocular lens 1 of this embodiment, multiple segmental regions 20 (20A, 20B, 20C) are formed on at least one of the front and rear surfaces. The multiple segmental regions 20 radiate outward from the center of lens section 2 and have different refractive powers due to different radii of curvature. In intraocular lens 1 of this embodiment, the multiple segmental regions 20 (in the example shown in FIG. 5, the multiple segmental regions 20 and transition regions 21) are arranged in lens section 2 so that the cross-sectional area of lens section 2 in first cross-section S1 is equal to or less than the cross-sectional areas in virtual cross-sections in other directions. Also, the multiple segmental regions 20 (in the example shown in FIG. 5, the multiple segmental regions 20 and transition regions 21) are arranged in lens section 2 so that the cross-sectional area of lens section 2 in second cross-section S2 is equal to or greater than the cross-sectional areas in virtual cross-sections in other directions. Therefore, unlike intraocular lenses with multiple regions arranged concentrically, even when the wearer's pupil becomes small, light passes through each of the multiple segmental regions 20 and focuses on the retina. Also, the multiple segmental regions 20 have different refractive powers due to different radii of curvature. Therefore, compared to forming microprism groups in segmental regions, the amount of light scattered in unintended directions decreases. As a result, loss of light reaching the retina is less likely to occur, and better vision is more easily obtained. Furthermore, intraocular lens 1 is folded small in the passage of the nozzle and inserted into the eye.

The technology disclosed in the above embodiment is merely an example. Therefore, it is possible to modify the technology exemplified in the above embodiment. First, it is possible to adopt only some of the multiple technologies exemplified in the above embodiment for intraocular lenses. For example, it is possible to adopt only the technology of forming three or more segmental regions 20 with different radii of curvature in lens section 2, without adopting the technology of reducing the cross-sectional area of lens section 2 in first cross-section S1. In this case, the intraocular lens may be inserted into the eye without using intraocular lens insertion instrument 100. Also, three or more segmental regions may be formed in intraocular lenses that do not have support sections. Even when three or more segmental regions are formed in intraocular lenses with support sections, the number and shape of support sections are not limited to those exemplified in the above embodiment.

Also, it is possible to adopt only the technology of reducing the cross-sectional area of lens section 2 in first cross-section S1, without adopting the technology of forming three or more segmental regions 20 in lens section 2. For example, the technology of reducing the cross-sectional area of lens section 2 in first cross-section S1 can be applied to toric intraocular lenses that correct the wearer's astigmatism. In this case, in toric intraocular lenses, the intraocular lens may be designed so that the position of the first cross-section coincides with the strong principal meridian of the lens section. Even in this case, the cross-sectional area of the lens section in the first cross-section becomes appropriately small.

Also, in the above embodiment, the expected movement direction D of intraocular lens 1 substantially coincides with extrusion axis A of intraocular lens insertion instrument 100 when front support section 3A of intraocular lens 1 contacts the inner wall of tapered nozzle 180. However, it is possible to change the method of making the movement direction of intraocular lens 1 substantially coincide with extrusion axis A of intraocular lens insertion instrument 100. For example, a guide or the like for making movement direction D of intraocular lens 1 substantially coincide with extrusion axis A may be separately provided.

Also, regardless of whether the MTF value conditions exemplified in the above embodiment are satisfied, it is possible to design intraocular lens 1 having multiple segmental regions to satisfy the conditions $S \geq +2.75$ D and $M < S \leq (M + 1.4$ D). Even in this case, by designing intraocular lens 1 to satisfy the conditions $S \geq +2.75$ D and $M < S \leq (M + 1.4$ D), vision at distance, near, and intermediate distances is even more easily obtained regardless of pupil size.

**Claims**

1. An intraocular lens, comprising:

   a disc-shaped lens section having a front surface and a rear surface, wherein
   at least one of the front and rear surfaces of the lens section is formed with three or more segmental regions having mutually different refractive powers,
   the three or more segmental regions include:

   a distance region with a smallest refractive power;
   a near region with a largest refractive power; and
   an intermediate region with a refractive power between the smallest refractive power and the largest refractive power, and

   the three or more segmental regions radiate outwardly from a center of the lens section.

2. The intraocular lens according to claim 1, wherein
   the three or more segmental regions have mutually different radii of curvature to have mutually different refractive powers.

3. The intraocular lens according to claim 1 or 2, wherein

   the lens section further includes a transition region formed between adjacent segmental regions of the three or more segmental regions, and
   the transition region has a radius of curvature changing from one of the adjacent segmental regions to the other of the adjacent segmental regions to smoothly connect ends of the adjacent segmental regions.

4. The intraocular lens according to claim 3, wherein
   both the segmental regions and the transition region refract light entering the lens section in a direction parallel to an optical axis of the lens section.

5. The intraocular lens according to claim 3 or 4, wherein
   a central angle of the transition region radiating outward from the center of the lens section is 5 degrees or more and 30 degrees or less.

6. The intraocular lens according to any one of claims 1 to 5, wherein

   each of central angles of the distance region, the near region, and the intermediate region has a central angle radiating outward from the center of the lens section, and
   the central angle of the distance region has a largest value and the central angle of the intermediate region has a smallest value.

7. The intraocular lens according to claim 6, wherein

the central angle of the distance region is 140 degrees or more,
the central angle of the near region is 90 degrees or more, and
the central angle of the intermediate region is 30 degrees or more.

8.  The intraocular lens according to any one of claims 1 to 7, wherein

    the refractive power of the near region is +2.5 D or more and +4.0 D or less, and
    the refractive power of the intermediate region is +1.0 D or more and +2.5 D or less.

9.  The intraocular lens according to any one of claims 1 to 8, wherein
    at least one of the front and rear surfaces of the lens section is formed with a toric surface for correcting astigmatism of a user.

10. The intraocular lens according to any one of claims 1 to 9, wherein

    a MTF curve at a spatial frequency of 50 lp/mm has an intermediate maximum, which is a maximum in a range of the MTF curve between a distance maximum and a near maximum, and
    each of the distance maximum, the near maximum, and the intermediate maximum is 0.10 or more.

11. The intraocular lens according to claim 10, wherein
    in the MTF curve at a spatial frequency of 50 lp/mm, a difference between a MTF value which is a smaller one of the near maximum and the intermediate maximum and a minimum value between the near maximum and intermediate maximum is 0.10 or less.

12. The intraocular lens according to claim 10 or 11, wherein
    in the MTF curve, the near maximum is greater than the intermediate maximum.

13. The intraocular lens according to any one of claims 10 to 12, wherein
    in the MTF curve at a spatial frequency of 50 lp/mm, a difference between the near maximum and the intermediate maximum is 0.05 or less.

14. The intraocular lens according to any one of claims 10 to 13, wherein

    the refractive power of the near region is S, and
    the refractive power of the intermediate region is M, wherein

$$S \geq +2.75 \text{ D and } M < S \leq (M + 1.4 \text{ D}).$$

15. The intraocular lens according to any one of claims 10 to 13, wherein

    the refractive power of the near region is S, and
    the refractive power of the intermediate region is M, wherein

$$S \geq +2.75 \text{ D and } (M + 1.1 \text{ D}) \leq S \leq (M + 1.4 \text{ D}).$$

16. The intraocular lens according to any one of claims 1 to 9, wherein

    the refractive power of the near region is S, and
    the refractive power of the intermediate region is M, wherein

$$S \geq +2.75 \text{ D and } M < S \leq (M + 1.4 \text{ D}).$$

# FIG. 1

LEFT SIDE

FRONT SIDE ← → REAR SIDE

RIGHT SIDE

# FIG. 2

UPPER SIDE

LEFT SIDE — REAR SIDE

FRONT SIDE — RIGHT SIDE

LOWER SIDE

# FIG. 3

UPPER SIDE

LEFT SIDE

REAR SIDE

FRONT SIDE

RIGHT SIDE

LOWER SIDE

# FIG. 4

# FIG. 5

LEFT SIDE

FRONT SIDE ← → REAR SIDE

RIGHT SIDE

# FIG. 6

# FIG. 7

# FIG. 8

# FIG. 9

# FIG. 10

## INTERNATIONAL SEARCH REPORT

International application No.

**PCT/JP2023/045486**

### A. CLASSIFICATION OF SUBJECT MATTER

***A61F 2/16*** (2006.01)i
FI: A61F2/16

According to International Patent Classification (IPC) or to both national classification and IPC

### B. FIELDS SEARCHED

Minimum documentation searched (classification system followed by classification symbols)

A61F2/16

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2012-517880 A (PROCORNEA HOLDING B.V.) 09 August 2012 (2012-08-09) paragraphs [0046], [0089]-[0099], [0102], [0110], fig. 9-11 | 1-7, 9 |
| Y | | 8, 10-16 |
| Y | JP 2020-535945 A (CRISTALENS INDUSTRIE) 10 December 2020 (2020-12-10) abstract, paragraphs [0005]-[0025], fig. 3 | 8, 10-16 |
| A | DE 202012102027 U1 (VR VISION RESEARCH GMBH) 26 June 2012 (2012-06-26) entire text, all drawings | 1 |
| A | JP 2010-125292 A (NIDEK CO., LTD.) 10 June 2010 (2010-06-10) entire text, all drawings | 1 |
| A | US 4798609 A (GRENDAHL, Dennis T.) 17 January 1989 (1989-01-17) entire text, all drawings | 1 |
| A | JP 2011-528272 A (ALCON, INC.) 17 November 2011 (2011-11-17) entire text, all drawings | 10-13 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 March 2024** | **19 March 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

| | International application No. |
|---|---|
| | **PCT/JP2023/045486** |

| Patent document cited in search report | | | Publication date (day/month/year) | Patent family member(s) | Publication date (day/month/year) |
|---|---|---|---|---|---|
| JP | 2012-517880 | A | 09 August 2012 | US 2012/0029631 A1 paragraphs [0044], [0113]-[0123], [0126], [0130], fig. 9-11 <br> GB 2483843 A <br> WO 2010/095938 A1 <br> EP 2219065 A1 <br> CA 2752794 A1 <br> KR 10-2011-0132381 A <br> CN 102395917 A | |
| JP | 2020-535945 | A | 10 December 2020 | US 2020/0281715 A1 abstract, paragraphs [0005]-[0025], fig. 3 <br> WO 2019/068645 A1 <br> FR 3072020 A1 <br> KR 10-2020-0069313 A <br> CN 111511311 A | |
| DE | 202012102027 | U1 | 26 June 2012 | (Family: none) | |
| JP | 2010-125292 | A | 10 June 2010 | (Family: none) | |
| US | 4798609 | A | 17 January 1989 | (Family: none) | |
| JP | 2011-528272 | A | 17 November 2011 | US 2010/0016965 A1 <br> WO 2010/009257 A1 <br> CA 2730123 A1 <br> KR 10-2011-0030696 A <br> CN 102099730 A | |

Form PCT/ISA/210 (patent family annex) (July 2022)

27

**EP 4 640 184 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010082290 A
- JP 2010125292 A